# EUROPEAN PATENT APPLICATION

(11) **EP 4 765 138 A1**
(43) Date of publication of application: **24.06.2026**
(21) Application number: 24222118.2
(22) Date of filing: 20.12.2024
(51) Int. Cl.: G16H 10/60, G16H 40/63, G16H 40/67

(54) **PATIENT COMMUNICATION DEVICE AND COMMUNICATION METHOD**

(71) Applicant: BIOTRONIK SE & Co. KG, 12359 Berlin (DE)
(72) Inventor: MOSS, Christian, 14612 Falkensee (DE); ELSNER, Joachim, 14059 Berlin (DE); WEGERICH, Franziska, 12055 Berlin (DE); BAUCELLS COSTA, Alejandro, 10405 Berlin (DE); SKERL, Olaf, 18209 Bad Doberan (DE); BODE, Sven, 10829 Berlin (DE); FINNBERG, Thomas, 22885 Barsbüttel (DE)
(74) Representative: Biotronik Corporate Services SE

(57) **Abstract**

The invention relates to a patient communication device (10), comprising means (11) for receiving patient medical data (12) and/or a machine-readable code (14), preferably a QR-Code, representing said patient medical data (12) or data for establishing a connection between the patient communication device (10) and a further communication device (15), optionally means (16) for generating the machine-readable code (14) based on the received patient medical data (12), and a graphical user interface (18) configured to display the machine-readable code (14) to be read by the further communication device (15), in particular a tablet or smartphone device. The invention further relates to a medical communication system (1) and a computer-implemented method for communication between a patient communication device (10) and a further communication device (15).

## Description

The invention relates to a patient communication device. Furthermore, the invention relates to a medical communication system comprising the patient communication device. In addition, the invention relates to a computer-implemented method for communication between a patient communication device and a further communication device.

Conventionally, a patient communication device is connected to a smartphone or tablet either directly via a wireless interface, e.g. Bluetooth or indirectly via the internet. Alternatively, a larger, higher-resolution display could be integrated into the patient communication device, i.e. a proprietary device.

A direct connection requires the patient communication device to be paired with the smartphone/tablet. An indirect connection also requires internet availability for both devices and an association between the devices. A display on the patient communication device would require a more expensive, larger and higher-resolution display.

Future telemedicine applications may include procedures that require the patient and clinical staff to interact with the patient communication device. The interaction should take place or should be carried out as quickly as possible and on site. However, a differentiated or detailed presentation of complex issues such as diagrams, instructions, etc. is only possible to a limited extent on a current patient communication device.

It is therefore an object of the present invention to provide an improved patient communication device and method for transferring information with a simple user interface to a smartphone/tablet without using a wireless connection.

The object is solved by a patient communication device having the features of claim 1.

Furthermore, the object is solved by a medical communication system having the features of claim 11.

In addition, the object is solved by a computer-implemented method for communication between a patient communication device and a further communication device having the features of claim 13.

Moreover, the object is solved by a computer program having the features of claim 14 and by a computer-readable data carrier having the features of claim 15.

Further developments and advantageous embodiments are defined in the dependent claims.

According to a first aspect, the present invention provides a patient communication device, comprising means for receiving patient medical data and/or a machine-readable code, preferably a QR-Code, representing said patient medical data or data for establishing a connection between the patient communication device and a further communication device.

The patient communication device optionally further comprises means for generating the machine-readable code based on the received patient medical data.

In addition, the patient communication device comprises a graphical user interface configured to display the machine-readable code to be read by the further communication device, in particular a tablet or smartphone device.

According to a second aspect, the present invention provides a medical communication system comprising the patient communication device according to the present invention.

The medical communication system further comprises an implantable medical device connected, in particular communicatively connected, to the patient communication device, wherein the implantable medical device is configured to send patient medical data and/or a machine-readable code to the patient communication device.

In addition, the medical communication system comprises a further communication device comprising an app, wherein the further communication device is configured to scan the machine-readable code displayed by the graphical user interface of the patient communication device.

For example, a machine-readable code is scanned by the app containing the request for confirmation that remote programming may take place.

Furthermore, using a smartphone/tablet offers the user a familiar operating environment for complex displays. The smartphone app can be configured user-specifically, i.e. the information of the machine-readable code is displayed by the app in a user-specific manner, e.g. patients differentiated by type of therapy and/or age, clinical staff differentiated by task, etc.

According to a third aspect, the present invention provides a computer-implemented method for communication between a patient communication device and a further communication device.

The method comprises receiving patient medical data and/or a machine-readable code, preferably a QR-Code, representing said patient medical data or data for establishing a connection between the patient communication device and a further communication device, generating the machine-readable code based on the received patient medical data, and displaying the machine-readable code to be read by the further communication device, in particular a tablet or smartphone device on a graphical user interface.

In addition, the present invention provides a computer program with program code to perform the method of the present invention when the computer program is executed on a computer. Moreover, the present invention provides a computer-readable data carrier containing program code of a computer program for performing the method of the present invention when the computer program is executed on a computer.

An idea of the present invention is to dispense with or avoid the use of wireless interfaces which represent added value in terms of compatibility. Conventionally, radio interfaces are constantly changing and are generally in a continuous standardization process. These changes are often linked to hardware changes. In the medium term, this leads to incompatibility between the patent communication device, which is a proprietary device and the smartphone/tablet. Using an alternative data transmission method by means of machine-readable code thus avoids or prevents the above-mentioned compatibility issues typically encountered with radio interfaces.

Furthermore, any changes to coding procedures can be made available to the patient communication device via software updates. The display of the patient communication device can be simple, e.g. black and white, robust, e.g. mechanically unbreakable, energy-saving, easy to control and small.

In a further aspect, it is proposed that the machine-readable code comprises all of the patient medical data to be transmitted to the further communication device. Thus, all relevant information can be transmitted via a single communication method.

In a further aspect, it is proposed that the machine-readable code comprises one or several code segments, wherein if a size of the patient medical data to be transmitted to the further communication device exceeds a data size of a code segment displayable on the graphical user interface, the patient medical data is transmittable to the further communication device in several consecutive code segments of machine-readable code. This advantageously enables transmission of patient medical data of any required size.

In a further aspect, it is proposed that the data for establishing the connection between the patient communication device and the further communication device comprises data for establishing a NFC or infrared connection, a URL and/or data for authenticating the connection between the patient communication device and the further communication device. The machine-readable code thus enables to control the further communication device in such a way that a connection between the patient communication device and the further communication device can be automatically established upon scanning the machine-readable code.

The machine-readable code can be used as an additional security in the case of remote programming an implantable medical device. By requiring the patient's smartphone/tablet to be in close proximity to or near the external device, it is thus possible to provide a form of two-factor authentication. Near or in close proximity to the external device means that the distance to the external device is a maximum of one meter, in particular a maximum of 20 cm.

In a further aspect, it is proposed that the patient medical data comprised by the machine-readable code or the data for establishing the connection between the patient communication device and the further communication device comprised by the machine-readable code is encrypted. The encrypted data is codified as machine-readable code which can in turn be decrypted in the app of the further communication device.

In a further aspect, it is proposed that the patient communication device is configured to signal receipt and/or an acknowledge of the patient medical data and/or the machine-readable code by generating a light and/or audio signal. This enables an effective way to notify the user of the availability of a message concerning new patient medical data.

In a further aspect, it is proposed that the machine-readable code is displayed on the graphical user interface only during a predetermined time period in which the patient medical data is clinically relevant. That way it is ensured that only relevant patient medical data is displayed at a given time.

In a further aspect, it is proposed that the patient communication device is configured to generate or receive the machine-readable code if the patient medical data fulfils predefined clinical criteria, in particular if a predefined medical condition is detected in the patient medical data. The algorithm or logic to detect the predefined medical condition or pattern in the patient medical data may run on a remote server which is configured to signal the patient communication device directly or via the further communication device once the predefined clinical criteria is met. But the algorithm or logic to detect the predefined medical condition or pattern in the patient medical data may also run on the patient communication device and/or the further communication device.

In a further aspect, it is proposed that the patient communication device is configured to receive a notification from the further communication device upon completing data transmission between the patient communication device and the further communication device, wherein the patient communication device is configured to display the machine-readable code until the data transmission between the patient communication device and the further communication device is completed. The machine-readable code is thus displayed before and during data transmission to the further medical device and automatically deleted after completion of the data transmission.

In a further aspect, it is proposed that the patient medical data comprises at least a segment of a patient ECG, preferably an intracardiac ECG recorded by an implantable medical device. The patient ECG is advantageously transmitted as time series data, wherein the app of the further medical device is configured to interpret and display said timeseries data in a suitable manner, e.g. at least in part graphically on a display of the further medical device.

The herein described features of the patient communication device are also disclosed for the computer-implemented method for communication between a patient communication device and a further communication device and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a diagram of a patient communication device according to a preferred embodiment of the invention;
- Fig. 2: shows a diagram of a medical communication system according to the preferred embodiment of the invention; and
- Fig. 3: shows a flowchart of a computer-implemented method for communication between a patient communication device and a further communication device.

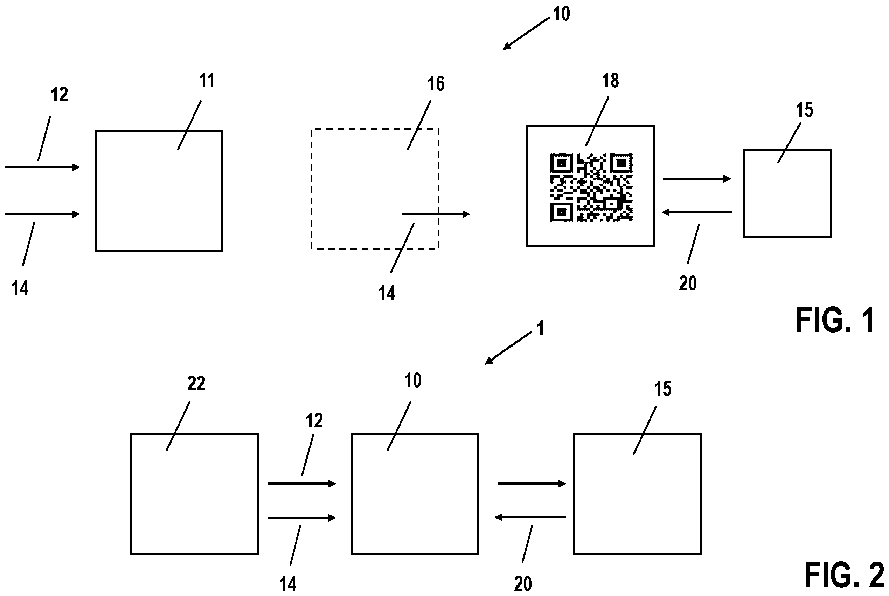

According to Fig. 1, the patient communication device 10 comprises means 11 for receiving patient medical data 12 and/or a machine-readable code 14, preferably a QR-Code, representing said patient medical data 12 or data for establishing a connection between the patient communication device 10 and a further communication device 15, optionally means 16 for generating the machine-readable code 14 based on the received patient medical data 12, and a graphical user interface 18 configured to display the machine-readable code 14 to be read by the further communication device 15, in particular a tablet or smartphone device.

The machine-readable code 14 comprises all of the patient medical data 12 to be transmitted to the further communication device 15.

Furthermore, the machine-readable code 14 comprises one or several code segments, wherein if a size of the patient medical data 12 to be transmitted to the further communication device 15 exceeds a data size of a code segment displayable on the graphical user interface 18, the patient medical data 12 is transmittable to the further communication device 15 in several consecutive code segments of machine-readable code 14.

In addition, the data for establishing the connection between the patient communication device 10 and the further communication device 15 comprises data for establishing a NFC or infrared connection, a URL and/or data for authenticating the connection between the patient communication device 10 and the further communication device 15.

Moreover, the patient medical data 12 comprised by the machine-readable code 14 or the data for establishing the connection between the patient communication device 10 and the further communication device 15 comprised by the machine-readable code 14 is encrypted. Alternatively, the machine-readable code 14 may not be encrypted.

The patient communication device 10 is configured to signal receipt of the patient medical data 12 and/or the machine-readable code 14 by generating a light and/or audio signal.

The machine-readable code 14 is displayed on the graphical user interface 18 only during a predetermined time period in which the patient medical data 12 is clinically relevant. Furthermore, the patient communication device 10 is configured to generate or receive the machine-readable code 14 if the patient medical data 12 fulfils predefined clinical criteria, in particular if a predefined medical condition is detected in the patient medical data 12.

In addition, the patient communication device 10 is configured to receive a notification 20 from the further communication device 15 upon completing data transmission between the patient communication device 10 and the further communication device 15, wherein the patient communication device 10 is configured to display the machine-readable code 14 until the data transmission between the patient communication device 10 and the further communication device 15 is completed. Moreover, the patient medical data 12 comprises at least a segment of a patient ECG, preferably an intracardiac ECG recorded by an implantable medical device.

Fig. 2 shows a diagram of a medical communication system 1 according to the preferred embodiment of the invention.

The medical communication system 1 comprises the patient communication device 10 according to the present invention. Furthermore, the medical communication system 1 comprises an implantable medical device 22 communicatively connected to the patient communication device 10, wherein the implantable medical device 22 is configured to send patient medical data 12 and/or a machine-readable code 14 to the patient communication device 10, and a further communication device 15 comprising an app, wherein the further communication device 15 is configured to scan the machine-readable code 14 displayed by the graphical user interface 18 of the patient communication device 10.

In addition, the patient medical data 12 represented by the machine-readable code 14 is time series data, wherein the app of the further communication device 15 is configured to process the time series data and generate at least on part a graphical output.

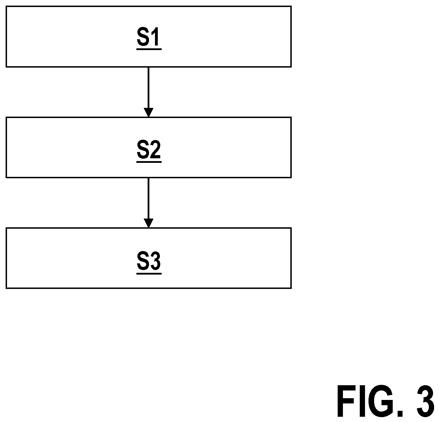

Fig. 3 shows a flowchart of a computer-implemented method for communication between a patient communication device 10 and a further communication device 15.

The method comprises receiving S1 patient medical data 12 and/or a machine-readable code 14, preferably a QR-Code, representing said patient medical data 12 or data for establishing a connection between the patient communication device 10 and a further communication device 15, generating S2 the machine-readable code 14 based on the received patient medical data 12, and displaying S3 the machine-readable code 14 to be read by the further communication device 15, in particular a tablet or smartphone device on a graphical user interface 18.

Although specific embodiments have been illustrated and described herein, it will be understood by those skilled in the art that a variety of alternative and/or equivalent implementations exist. It should be noted that the exemplary embodiment or exemplary embodiments are examples only and are not intended to limit the scope, applicability or configuration in any way.

Rather, the foregoing detailed description provides the skilled person with a convenient guide to implementing at least one exemplary embodiment, it being understood that various changes in the scope of functionality and arrangement of the elements may be made without departing from the scope of the appended claims and their legal equivalents.

In general, this application intends to cover modifications or adaptations or variations of the embodiments disclosed herein. For example, a sequence of method steps may be modified. The method may further be carried out sequentially or in parallel, at least in part.

### Reference Signs

- 1: Medical communication system
- 10: Patient communication device
- 11: means for receiving patient medical data
- 12: patient medical data
- 14: machine-readable code
- 15: further communication device
- 16: means for generating the machine-readable code
- 18: graphical user interface
- 20: notification
- 22: implantable medical device
- S1-S3: method steps

## Claims

1. Patient communication device (10), comprising:
means (11) for receiving patient medical data (12) and/or a machine-readable code (14), preferably a QR-Code, representing said patient medical data (12) or data for establishing a connection between the patient communication device (10) and a further communication device (15);
optionally means (16) for generating the machine-readable code (14) based on the received patient medical data (12); and
a graphical user interface (18) configured to display the machine-readable code (14) to be read by the further communication device (15), in particular a tablet or smartphone device.

2. Patient communication device of claim 1, wherein the machine-readable code (14) comprises all of the patient medical data (12) to be transmitted to the further communication device (15).

3. Patient communication device of claim 1 or 2, wherein the machine-readable code (14) comprises one or several code segments, wherein if a size of the patient medical data (12) to be transmitted to the further communication device (15) exceeds a data size of a code segment displayable on the graphical user interface (18), the patient medical data (12) is transmittable to the further communication device (15) in several consecutive code segments of machine-readable code (14).

4. Patient communication device of claim 1, wherein the data for establishing the connection between the patient communication device (10) and the further communication device (15) comprises data for establishing a NFC or infrared connection, a URL and/or data for authenticating the connection between the patient communication device (10) and the further communication device (15).

5. Patient communication device of any one of the preceding claims, wherein the patient medical data (12) comprised by the machine-readable code (14) or the data for establishing the connection between the patient communication device (10) and the further communication device (15) comprised by the machine-readable code (14) is encrypted.

6. Patient communication device of any one of the preceding claims, wherein the patient communication device (10) is configured to signal receipt of the patient medical data (12) and/or the machine-readable code (14) by generating a light and/or audio signal.

7. Patient communication device of any one of the preceding claims, wherein the machine-readable code (14) is displayed on the graphical user interface (18) only during a predetermined time period in which the patient medical data (12) is clinically relevant.

8. Patient communication device of any one of the preceding claims, wherein the patient communication device (10) is configured to generate or receive the machine-readable code (14) if the patient medical data (12) fulfils predefined clinical criteria, in particular if a predefined medical condition is detected in the patient medical data (12).

9. Patient communication device of any one of the preceding claims, wherein the patient communication device (10) is configured to receive a notification (20) from the further communication device (15) upon completing data transmission between the patient communication device (10) and the further communication device (15), wherein the patient communication device (10) is configured to display the machine-readable code (14) until the data transmission between the patient communication device (10) and the further communication device (15) is completed.

10. Patient communication device of any one of the preceding claims, wherein the patient medical data (12) comprises at least a segment of a patient ECG, preferably an intracardiac ECG recorded by an implantable medical device.

11. Medical communication system (1) comprising:
the patient communication device (10) of any one of claims 1 to 10;
an implantable medical device (22) communicatively connected to the patient communication device (10), wherein the implantable medical device (22) is configured to send patient medical data (12) and/or a machine-readable code (14) to the patient communication device (10); and
a further communication device (15) comprising an app, wherein the further communication device (15) is configured to scan the machine-readable code (14) displayed by the graphical user interface (18) of the patient communication device (10).

12. Medical communication system of claim 11, wherein the patient medical data (12) represented by the machine-readable code (14) is time series data, wherein the app of the further communication device (15) is configured to process the time series data and generate at least on part a graphical output.

13. Computer-implemented method for communication between a patient communication device (10) and a further communication device (15), comprising the steps of:
receiving (S1) patient medical data (12) and/or a machine-readable code (14), preferably a QR-Code, representing said patient medical data (12) or data for establishing a connection between the patient communication device (10) and a further communication device (15);
generating (S2) the machine-readable code (14) based on the received patient medical data (12); and
displaying (S3) the machine-readable code (14) to be read by the further communication device (15), in particular a tablet or smartphone device on a graphical user interface (18).

14. Computer program with program code to perform the method of claim 13 when the computer program is executed on a computer.

15. Computer-readable data carrier containing program code of a computer program for performing the method of claim 13 when the computer program is executed on a computer.
